# EUROPEAN PATENT SPECIFICATION

(11) **EP 3 305 901 B1**
(45) Date of publication and mention of the grant of the patent: **30.09.2020**
(21) Application number: 15893474.5
(22) Date of filing: 07.12.2015
(51) Int. Cl.: C12N 15/12, C12N 15/63, A61K 48/00, A61P 9/00, A61P 19/00, A61P 21/00

(54) **OPTIMIZED NUCLEOTIDE SEQUENCE AND PHARMACEUTICAL COMPOSITION BASED THEREON WITH PROLONGED VEGF TRANSGENE EXPRESSION**
OPTIMIERTE NUKLEOTIDSEQUENZ UND DARAUF BASIERENDE PHARMAZEUTISCHE ZUSAMMENSETZUNG MIT VERLÄNGERTER VEGF-TRANSGEN-EXPRESSION
SÉQUENCE NUCLÉOTIDIQUE OPTIMISÉE ET COMPOSITION PHARMACEUTIQUE SUR SA BASE AVEC UNE EXPRESSION PROLONGÉE DU TRANSGÈNE VEGF

(30) Priority: 26.05.2015 RU 2015119768
(43) Date of publication of application: 11.04.2018
(73) Proprietor: "Nextgen" Company Limited, Moscow 119333 (RU)
(72) Inventor: KISELEV, Sergey Lvovich, Moscow 123458 (RU)
(74) Representative: Glawe, Delfs, Moll
(86) International application number: PCT/RU2015/000852
(87) International publication number: WO 2016/190774

(56) References cited:
- WO-A1-2005/095615
- WO-A1-2014/035289
- US-A- 5 587 300
- MIN DU ET AL: "VEGF gene expression is regulated post-transcriptionally in macrophages", FEBS JOURNAL, vol. 273, no. 4, 1 February 2006 (2006-02-01), pages 732-745, XP055505798, GB ISSN: 1742-464X, DOI: 10.1111/j.1742-4658.2006.05106.x
- DATABASE EMBL [Online] 11 December 2006 (2006-12-11), "CC-F01_006_K07 Cherry of different development stage Coffea canephora cDNA clone CC-F01_006_K07, mRNA sequence.", XP002784565, retrieved from EBI accession no. EM_EST:EE196866 Database accession no. EE196866
- ARCONDEGUY TANIA ET AL: "VEGF-A mRNA processing, stability and translation: a paradigm for intricate regulation of gene expression at the post-transcriptional level", NUCLEIC ACIDS RESEARCH, vol. 41, no. 17, September 2013 (2013-09), pages 7997-8010, XP002784566,
- ANA M ZUBIAGA ET AL: "The Nonamer UUAUUUAUU Is the Key AU-Rich Sequence Motif That Mediates mRNA Degradation", MOLECULAR AND CELLULAR BIOL, AMERICAN SOCIETY FOR MICROBIOLOGY, WASHINGTON, US, vol. 15, no. 4, 1 April 1995 (1995-04-01), pages 2219-2230, XP008164595, ISSN: 0270-7306, DOI: 10.1128/MCB.15.4.2219
- SHVALB P.G. ET AL.: '«Effektivnost i bezopasnost primeneniya preparata ''Neovaskulgen'' v kompleksnoi terapii patsientov s khronicheskoi ishemiei nizhnikh konechnostei (IIb-III faza klinicheskikh ispytanii' KLETOCHNAYA TRANSPLANTOLOGIYA I TKANEVAYA INZHENERIYA vol. VI, no. 3, 2011, pages 76 - 83, XP009504673

## Description

### TECHNICAL FIELD

The invention relates to bioengineering, more particularly, to developing of optimized preparations for gene therapy having a high stability and prolonged VEGF transgene expression.

### RELATED ART

### Preparations for gene therapy

Preparations for gene therapy, i.e., a group of pharmaceuticals whose active substance comprises gene constructs, namely, nucleic acids containing one or more genes coding for therapeutic proteins, are becoming of ever greater importance in the modern medicine.

Until now, five such preparations have been already registered and introduced in clinical practice and hundreds are undergoing experimental studies and clinical trials. The total number of gene therapy clinical trials conducted since 1989 exceeds 1900 [1]. One of the five registered preparations for gene therapy, Neovasculgen was developed and introduced in clinical practice in the territory of Russia (Reg. Certificate No. LP-000671 of 28.09.11) and Ukraine (Reg. Certificate No. 899/13300200000 of 25.01.2013) by the present Applicants (Human Stem Cells Institute PJSC).

Therefore, a principally new class of pharmaceutical preparations, namely, medicaments for gene therapy has come into being with the progress of bioengineering.

A plasmid DNA belongs to the safest variations of gene constructs carrying a transgene. It is the plasmid DNA with a VEGF gene that is the active substance of the above-mentioned Neovasculgen preparation for gene therapy. As recognized by absolute majority of researchers, the most serious problem associated with the plasmid DNA as a vector for transgene delivery to the target cells is a low transfection efficiency: only 1-2% of the total number of gene construct molecules reach the cells and/or are expressed by the same [2]. At the same time, the number of the plasmid DNA molecules having reached the cell and the total number of transfected cells is one of the major factors directly determining the production level of therapeutic protein and, accordingly, the biological effect of the medicament. In this connection, in view of the evolution of preparations for gene therapy as a new pharmacological class, it is highly topical to develop methods for increasing the production of therapeutic protein coded by the gene construct along with reducing the amount of the preparation being administered or improving the efficiency of its effect. It is particularly important for the plasmid DNA whose transfection efficiency is extremely low.

The total therapeutic protein concentration may be increased by applying two principal approaches. The most obvious first approach consists in increasing the transfection level of gene constructs. In other words, the more plasmid DNA reaches the target cells, the higher the concentration of the protein coded thereby will be produced. A number of physical and chemical methods have been proposed in the context of this approach. However, physical methods for increasing the transfection level require special equipment and are not always safe and feasible in clinical practice. At the same time, chemical methods require modification of the compositions of preparations for gene therapy, which call for additional production resources and may also be associated with negative effects of added components.

The second alternative approach consists in extending the lifetime of the transcription product of the administered gene construct, i.e. mRNA, resulting in an increased number of translation cycles and, accordingly, an increased amount of the therapeutic protein due to its prolonged production. The fundamentals of this approach have been formed as a result of accumulation of information about how the mRNA lifetime is regulated. Thus, a number of regulatory molecules are described, involved in modulating the mRNA stability, such as RNA-binding proteins and regulatory RNAs (microRNAs, long noncoding RNAs). Importantly, the effect of said substance on the mRNA is mediated via binding to its 3'-untranslated region (3'UTR) comprising special sequences as destabilizing elements, in particular adenine-uridine-rich element (AU-rich element). Some substances, such as AU-rich element RNA-binding protein 1 (AUF1), tristetraprolin (TTP), KH-type splicing regulatory protein (KSRP) induce mRNA degradation by binding to 3'UTR specific sites in contrast with other substances, for example, polyadenylate-binding protein-interacting protein 2 (PAIP2) which enables mRNA stabilization [3, 4].

It is important for normal cell functioning to coordinate the processes of mRNA degradation and stabilization, and according to a number of researchers, some pathological conditions of inflammatory or oncologic genesis may be associated with post-transcriptional dysregulation leading either to an insufficient or excessive production of growth factors, oncogenes and other biologically active substances [4]. Accordingly, the 3'UTR nucleotide sequence is of principal importance for transducing the regulatory impacts whose effector is mRNA and for determining the mRNA lifetime as a whole.

As follows from the analysis of the state of the art, modifying the 3'UTR nucleotide sequence, in particular destabilizing elements may enable extension of the mRNA lifetime. Moreover, methods have been developed for site-specific mutagenesis, allowing the necessary changes in the gene construct and, accordingly, in mRNA as its transcription product to be carried out technically. However, until now, no exhaustive and systemized information is available as to which variants of the 3'UTR sequence, which minor sites thereof or their combinations are responsible for which aspects of the mRNA functioning [3]. It is partly due to that the mRNA isoforms differing in their 3'UTR nucleotide sequence vary within a wide range because of, but not limited to, alternative splicing and polyadenilation. At the same time, the composition of 3'UTR destabilizing elements is determined qualitatively and quantitatively also by the nucleic acid coding region. All that in combination with the fact that 3'UTR determines not only the mRNA lifetime but also other constituents of its "physiology", such as cell nucleus transport, cytoplasmic localization, translation efficiency, etc., accounts for the difficulties and largely unpredictability of the mRNA lifetime modeling via changing the 3'UTR sequence, which may manifest itself both as an increase and a decrease in the transcript lifetime.

Despite all difficulties and insufficiently studied issues of determination or "programming" of the mRNA lifetime via changing the 3'UTR sequence, attempts are still being made to select empirically such variants of changes in the respective region of specific gene construct variants that would enhance the mRNA stability without negatively affecting its functioning.

In particular, known is a method for increasing transgene production by substituting the AU-rich element sequence presented by AUUUA with other variants and combinations thereof limited to AUGUA, AUAUA, GUGUG, AGGGA, GAGAG [5]. However, the above sequence of the destabilizing elements in 3'UTR is specific to far from all genes (described by most of researchers for G-CSF) and, on the other hand, it does not exhaust the list of destabilizing elements so that the mRNA lifetime extension may be leveled.

A special 3'UTR sequence of the erythropoetin gene has been also developed, enabling a prolonged production of the transgene being part of the plasmid DNA. The sequence is strictly specific and has a length of 100 nucleotides [6].

Methods for elimination of specific 3'UTR sequences responsible for binding to various microRNAs inducing the mRNA degradation have been also described [7].

However, most of the proposed changes in the 3'UTR sequence relate to lengthy deletions or substitutions, which is inevitably associated with the risk of negative effects on the mRNA metabolism. In addition, the prior art solutions are highly dependent on the gene coding region so that some of such solutions are inapplicable while other solutions are insufficiently efficient for the VEGF gene and extension of the lifetime of its transcription product.

In contrast, it is an object of the present invention to increase the VEGF production by target cells (for example, HEK293 human cell line, multipotent mesenchymal stromal cells, human fibrolasts and others) in which a plasmid DNA has penetrated by extending the lifetime of the mRNA specific to said gene by means of optimizing its 3'UTR.

### VEGF and its biological role

VEGF (vascular endothelial growth factor) is a family of biologically active proteins first isolated by J. Folkman et al. in 1971 [8], considered to be one of major auto- and para-crine factors of regulation of vasculo-, angio- (VEGF-A, B; PIGF) and lymphogenesis (VEGF-C, D); it is produced by the cells of all body tissues including epithelial tissues.

The medical use of VEGF is known from WO 2014/035289 A1 and Min Du et al., FEBS Journal 273 (2006), pages 732-745 discloses luciferase reporter expression constructs containing either the proximal or the distal region of 3'UTR of VEGF mRNA.

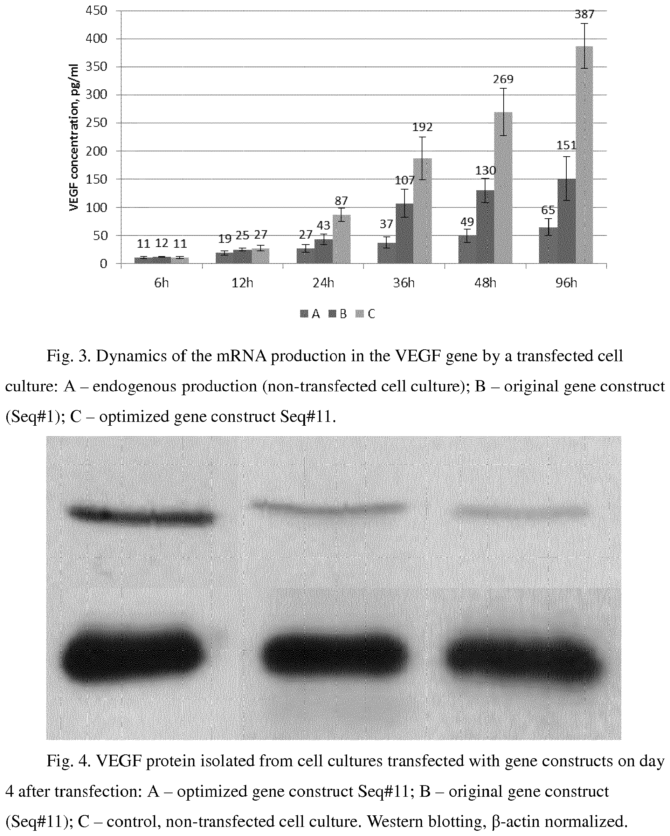

It is VEGF-A (isoforms 121, 145, 148, 165, 183, 189, 206) [9] that influences mostly the formation of vasculature during postnatal development of a human. Three types of VEGF receptors have been identified. Types 1 and 2 are involved in angiogenesis, and type 3 - in the formation of lymphatic vessels. At the same time, while type 1 receptor has a higher affinity to the VEGF, its tyrosine kinase activity is much lower than that of type 2 receptor, which is regarded as one of the regulatory mechanisms preventing an excessive VEGF activity. Accordingly, it is through type 2 receptor that the VEGF effects are normally realized [10, 11]. Upon VEGF interaction with specific type 2 receptor, autophosphorilation of its intracellular tyrosine sites (Y951, 1054, 1059, 1175, 1214) of kynase and carboxyterminal domains takes place [11], which in turn activates a number of intracellular proteins, such as phospho-lipases Cγ, Cβ3, SRK, NCK, SHB, SCK adaptor proteins and others, which comprise the first units of signal transduction complex cascades modifying the morphofunctional state of target (mostly endothelial) cells. In particular, phospholipase Cγ hyrdolizes the PIP₂ membrane phospholipid resulting in the formation of diacylated glycerol and inositol-1,4,5-triphosphate increasing the intracellular content of calcium, which together activates protein kinase C which in turn triggers sequential activation of the RAS-ERK signal path leading to the induction of mitosis. As a result, the proliferative activity of endothelial cells becomes higher [10]. Phospholipase Cβ3 is involved in the polymerization of actin and formation of stress fibrils enabling migration and motor performance of cells in general [12]. VEGF blocks the apoptosis via activation of the "phosphoinositide-3-kinase - protein kinase B" (PI3K/AKT) signal path, inhibiting caspases 3, 7 and 9 and thereby increasing the cell survival rate. In addition, the PI3K/AKT axis modulates, with the help of calcium ions, the activity of endothelial NO-synthase, which is accompanied by an increased NO production and an increased vascular permeability being a necessary part of angiogenesis (Fig. 3) [13, 14]. Therefore, the VEGF, via specific type 2 receptor, induces activation, migration and differentiation of endotheliocytes and their precursor cells, increased cell survival rate, which in combination with modulating the intercellular interactions and increasing the vascular permeability provides a prerequisite for the formation of capillary-like constructs followed by their remodeling into "mature" vessels [10-16].

Considering the role of VEGF as a key angiogenic factor, variant genes coding the same are used for developing gene constructs suitable for treating patients with cardiovascular diseases of ischemic genesis [17, 18]. In addition, non-specific angiogenic effect of such preparations for gene therapy may have a positive influence in case of other pathological conditions requiring activation of a reparative process: injury of peripheral nerves [19], diabetic foot syndrome [20], amyotrophic lateral sclerosis [21], injuries of skeleton bones [22, 23] and others.

As to the bone tissue, both in case of primary and secondary osteohistogenesis, precisely the vessels growing into the loose fibrous connective or cartilaginous tissues create the required conditions for differentiation of the resident cells in the osteoblastic direction as well as for migration of cambial reserves (perivascularly and with the blood flow). Apart from angiogenesis-mediated influence, VEGF also has a direct stimulating effect on the osteoblastic programmed differentiation cells which not only produce VEGF [24] but also express its types 1 and 2 receptors both during embryogenesis [25] and postnatal development [26]. It has been shown that the VEGF enables a considerable increase (up to 70%) in the proliferation of the osseous tissue cambial cells and also activates migration of the osteogenic cells according to the VEGF concentration gradient [27-29].

In the past few years, a principally different mechanism referred to as "intracrinic" has been identified apart from the canonical receptor mechanism of the VEGF action. These findings were confirmed by the studies having shown that the progenitor cells committed in the osteoblastic direction (expressing Osx) synthesize the VEGF not only "for exportation" but also to enable its own differentiation in the osteoblastic direction [30].

Therefore, the VEGF has a wide spectrum of action on the endothelial and mesenchymal cell lines. Although the main biological effect of the VEGF is associated with inducing the formation of blood and lymphatic vessels, other mechanisms of direct action on the cells of various programmed differentiations via receptor and intracrinic mechanisms are also seemingly present in the VEGF.

In this connection, a plasmid DNA with the VEGF gene, characterized by a prolonged transgene expression would become an efficient tool for developing medicinal preparations and gene-activated medical products intended for treating patients not only with cardiovascular diseases but also with other pathology, in which case a local increase in the VEGF level within the affected area would enable an enhanced reparative process.

### BRIEF DESCRIPTION OF THE DRAWINGS

Fig. 1 shows a dynamics of mRNA production (accumulation) by cell cultures transfected with variant gene constructs: 1 - Seq#1 (original gene construct), 2 - Seq#2, 3 - Seq#3, 4 - Seq#4, 5 - Seq#5, 6 - Seq#6, 7 - Seq#7, 8 - Seq#8, 9 - Seq#9, 10 - Seq#10, 11 - Seq#11.
Fig. 2 shows a dynamics of the mRNA production (accumulation) in the VEGF gene by a transfected cell culture: A - original gene construct (Seq#1); B - optimized gene construct SEQ#11. The results are presented as a multiple magnification in comparison with the mRNA production level in the endogenous VEGF.
Fig. 3 shows a dynamics of the mRNA production in the VEGF gene by a transfected cell culture: A - endogenous production (non-transfected cell culture); B - original gene construct (Seq#1); C - optimized gene construct Seq#11.
Fig. 4 shows the VEGF protein isolated from cell cultures transfected with gene constructs on day 4 after transfection: A - optimized gene construct Seq#ll; B - original gene construct (Seq#11); C - control, non-transfected cell culture. Western blotting, β-actin normalized.
Fig. 5 shows a change in the blood flow perfusion rate in an ischemic limb at different observation times: -- Non ChLI control, -- ChLI non treated, -- water for injection,-Seq#11 (200 mkg × 1 and 14 d), -- Seq#11 (100 mkg × 14 d).
Fig. 6 shows an ischemic muscle 35 days after surgery and administration of: A - water for injection, B - Seq#11 (200 mkg × 1 and 14 d).
Fig. 7 shows CD34⁺-cells/muscle fibers ratio. 1 - non ChLI control; 2 - ChLI non treated; 3 - water for injection; 4 - Seq#11 (100 mkg × 14 d).
Fig. 8 shows defects in the parietal bones of rabbits 30 days after implantation of osteoplastic materials: A - calcium phosphate with plasmid DNA Seq#ll, B - calcium phosphate without plasmid DNA.

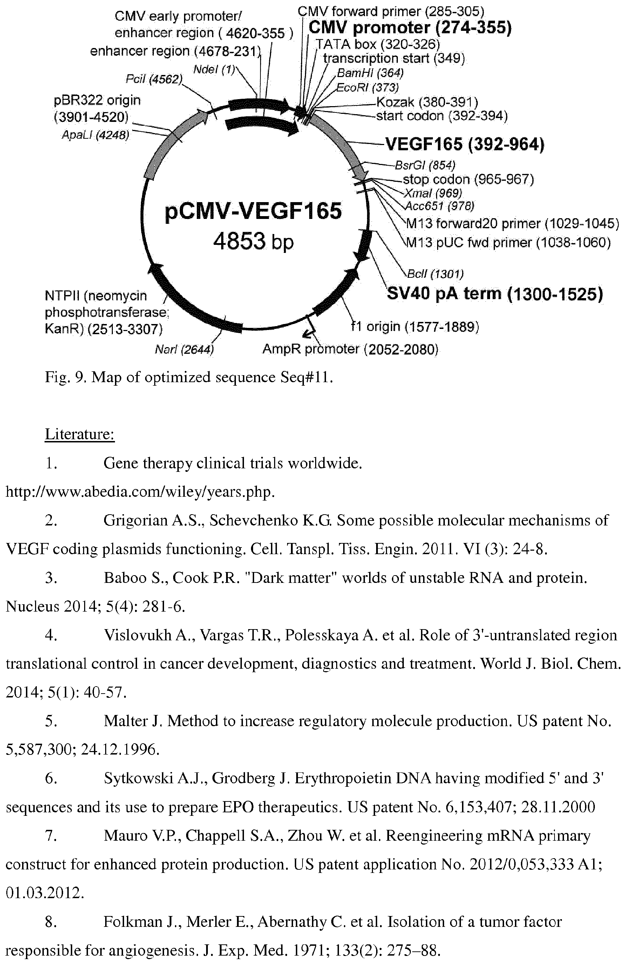

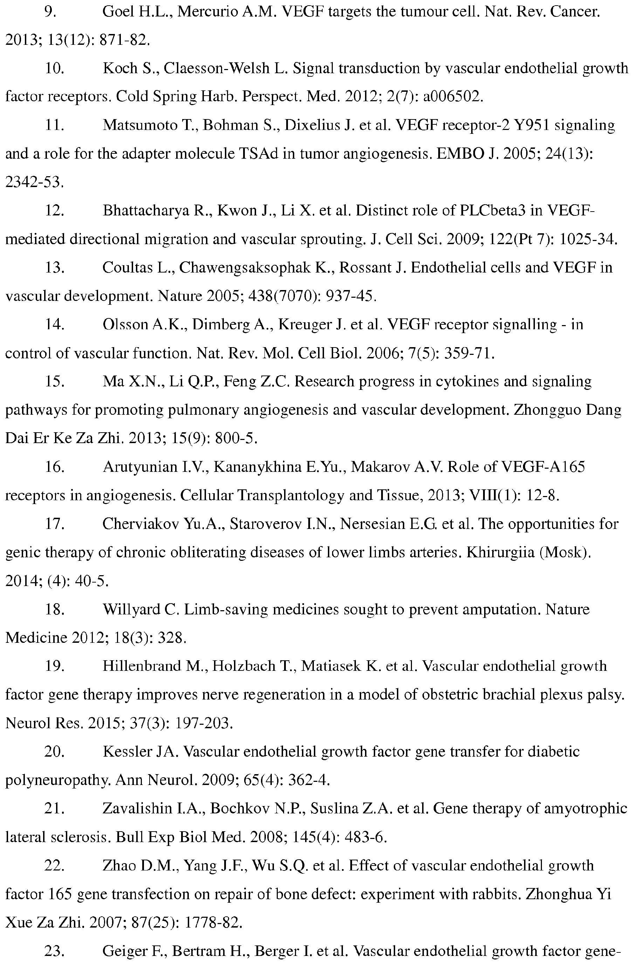

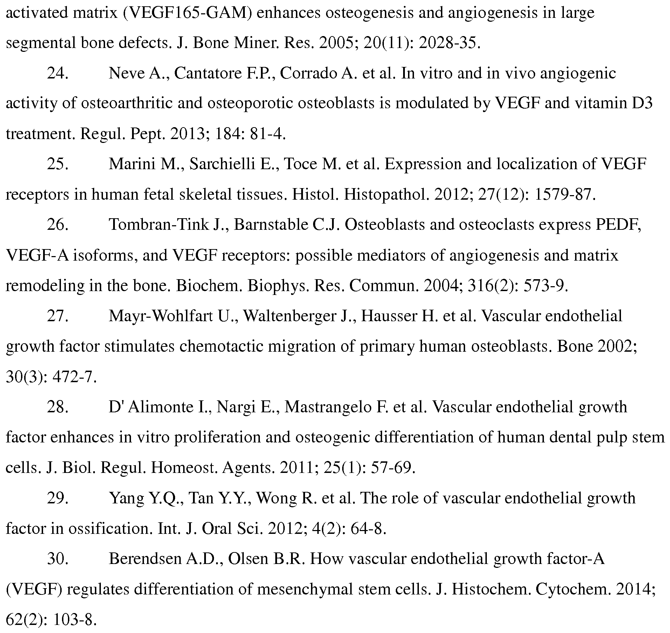

Fig. 9 shows a map of optimized sequence Seq#11.

### SUMMARY OF THE INVENTION

The invention is set out in the appended claims. The embodiments of the description which do not fall within the scope of said claims are provided for illustrative purposes only and do not form part of the present invention.

By the methods of site-specific mutagenesis and quantitative PCR analysis of the RNA molecules in a cell, the Applicants have studied the constructs with different changes in the 3'UTR nucleotide sequence of the therapeutic VEGF gene in comparison with the original gene construct (Seq#1, previously developed by the Applicants). Table 1 below shows the exemplary influences of some changes. As can be seen from the provided information, some changes in the nucleotide sequence (deletions or substitutions) positively affected the lifetime (for example, deletion of C 1079) while other changes had no effect (for example, deletion of C 1100) or led to a decrease in the mRNA lifetime (for example, deletion of C 1090). At the same time, in extreme cases (deletion of C 1079 - increase; deletion of C 1090 - decrease), the mRNA lifetime in VEGF gene varied by more than 6 hours at average with the mRNA average lifetime being of 6 hours.

At the second stage, all changes having a positive influence were used for developing a number of gene construct variants with four to ten "positive" mutations in various combinations, i.e. differing in the nucleotide sequence in the interval of 1070-1600 (Seq#2-11).

Based on these findings, the Applicants selected and synthesized an original 3'UTR terminal part of the VEGF gene, which in combination with the gene coding region enabled a increase by 70% in the gene transcript lifetime (SEQ#11), and wherein some mutations having the most positive influence in the aggregate were brought together, namely: deletion of C 1079, deletion of T 1111, substitution of A with C 1144, deletion of A 1148, deletion of C 1155, deletion of A 1173, deletion of C 1185, substitution of G with C 1536.

The resulting gene construct variant is characterized by a maximum mRNA lifetime and, accordingly, highest total production of the VEGF protein, which exceeds the production typical for the original gene construct (Seq#1).

Based on the developed optimized gene construct and adjuvants enabling cryoprotection, pH stabilization and preparation of an isotonic solution for injections, pharmaceutical compositions with a marked angiogenic activity were produced.

The resulting pharmaceutical compositions are suitable for use in case of diseases and pathological conditions the treatment of which requires stimulation of angiogenesis (tissue ischemia) or reparative tissue regeneration which may be accomplished through activation of angiogenesis (synthesis of continuity of peripheral nerves, bones, etc.)

For producing the developed pharmaceutical compositions, a bacterial (E.colli) producer strain was created for gene construct Seq#11, deposited at the All-Russian Collection of Microorganisms in accordance with the Budapest Treaty on the International Recognition of the Deposit of Microorganisms for the Purposes of Patent Procedure (strain deposit number VKM B-2967D). Attached hereto is the Certificate of the Strain International Deposit.

### Detailed Description

The site-specific mutagenesis of the VEGF gene 3'UTR was accomplished using the QuikChange Lightning Site-Directed Mutagenesis Kit (Agilent Technologies, USA) according to the manufacturer's instructions.

The site-specific mutagenesis of the original plasmid DNA (Seq#1) resulted in variant changes in the 3'UTR of VEGF gene. The resulting plasmid DNA variant with the VEGF gene differing in the 3'UTR sequence were used for transfection of the HEK293 cell lines followed by determination of the dynamics of accumulation of the gene construct expression product, i.e. mRNA of the VEGF gene.

**Table 1 - Examples of some changes in the nucleotide sequence and their influence on the mRNA lifetime**

| Position* | Change | Original nucleotide | Corrected nucleotide | Influence on the mRNA lifetime |
|---|---|---|---|---|
| 1071 | Deletion | C | - | increase |
| 1079 | Deletion | C | - | increase |
| 1090 | Deletion | C | - | decrease |
| 1100 | Deletion | C | - | no influence |
| 1111 | Deletion | T | - | increase |
| 1113 | Substitution | G | C | no influence |
| 1144 | Substitution | A | C | increase |
| 1146 | Deletion | G | - | decrease |
| 1148 | Deletion | A | - | increase |
| 1150 | Deletion | C | - | no influence |
| 1155 | Deletion | C | - | increase |
| 1158 | Deletion | C | - | decrease |
| 1173 | Deletion | A | - | increase |
| 1175 | Deletion | C | - | decrease |
| 1183 | Deletion | G | - | increase |
| 1184 | Deletion | C | - | decrease |
| 1185 | Deletion | C | - | increase |
| 1187 | Substitution | A | C | no influence |
| 1536 | Substitution | G | C | increase |

| | | | | |
|---|---|---|---|---|
| * Note: nucleotide numbering is given according to the original reference sequence of gene construct Seq#1. | | | | |

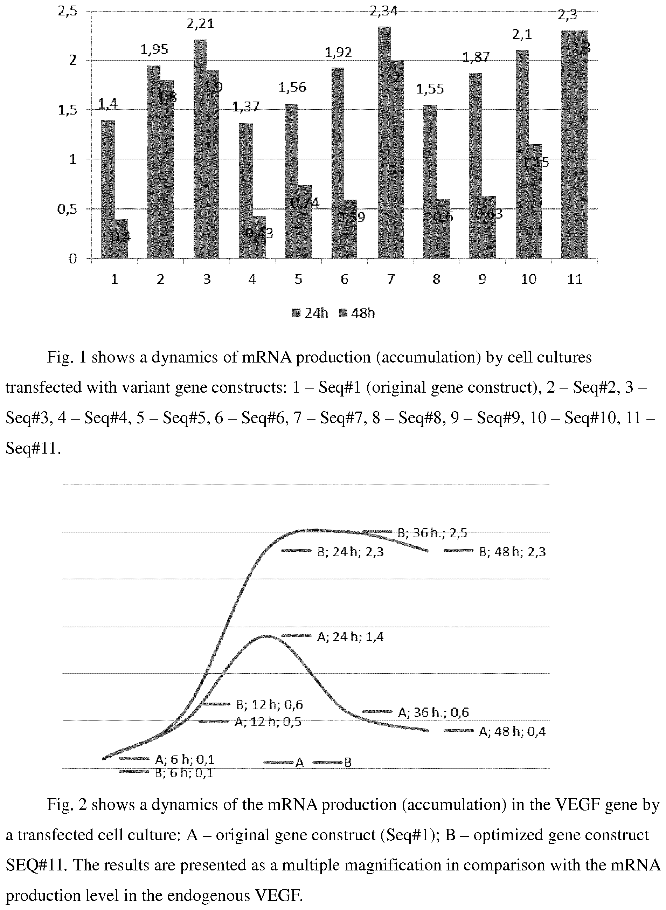

To this end, 2×10⁵ of HEK293 cells were placed onto the wells of a 6-well plate containing DMEM/F-12 cultural medium with added 10% FBS. After attachment of the cells, 10 µg of one of the obtained plasmid DNA variants was added to the cultural medium (in 100 µl of water for injection). As a negative control, 100 µl of water for injection without plasmid DNA was used so that the production level of the endogenous VEGF's mRNA could be assessed. In order to determine the VEGF gene expression level, the real-time PCR was accomplished for isolating total RNA from the cells 6, 12, 24, 36 and 48 hours after transfection by a column-based method using PureLink RNA Mini Kit (Invitrogen, USA) according to the manufacturer's instruction. Briefly, the precipitated cells were carefully lysed in 350 µl of a lytic buffer in the presence of 1% β-mercaptoethanol. An equal volume of 70% ethanol was added, vortexed and transferred onto the columns, centrifuged for 1 min at 12,400 rpm. The centrifuge liquid was drained. 600 µl of washing solution 1 was added onto the membrane, centrifuged for 15 sec at 12,400 rpm, and the liquid was removed. 500 µl of washing solution 2 was added onto the membrane, centrifuged under the same conditions, and the liquid was removed. The washing procedure was repeated using washing solution 2. The columns were transferred into new vials and centrifuged for 1 min at 12,400 rpm. The columns were transferred into vials to collect RNA. 15 µl of water without RNAs was added directly onto the membrane, incubated for 1 min at room temperature, and centrifuged for 90 sec at 12,400 rpm. The samples were kept at -80°C until further use. Then, a first chain of complementary DNA was synthesized using the reagents available from Promega (USA). The expression level of target genes (in moles) was determined as compared to the expression of β-actin housekeeping gene using a kit for accomplishing the real-time PCR in the presence of SYBR Green I and ROX reference colorant (Syntol, Russia). Fig. 1 shows an example of a nucleotide substitution enabling an increase in the mRNA lifetime.

Reverse transcription reaction:
1) on ice: 2.5 µl of Random Hexamer was added to 10 µl of RNA and incubated for 5 min at 70°C;
2) ice was removed;
3) drops were settled;
4) a mixture was prepared for each sample, comprising:
   - 5 µl of MMLV 5X reverse transcriptase buffer
   - 1.25 dNTP
   - 1.25 RNase Inhibitor
   - 1 µl of MMLV transcriptase
   - 4 µl of H₂O
5) 12.5 µl of the mixture was added to each vial and incubated for 1 hour at 37°C;
6) reactions were stopped by incubation at 75°C for 5 min.

All complementary DNA samples were kept at -80°C.

Forward and reverse primers for VEGF and β-actin were used to accomplish the real-time PCR (Table 2). The real-time PCR reaction mixture was used in the presence of SYBR Green I according to the manufacturer's instructions. The measurements were carried out using CFX90 Touch Real-time PCR Detection System.

**Table 2 - PCR Primers**

| Gene | Forward primer | Reverse primer |
|---|---|---|
| VEGF | ACATTGTTGGAAGAAGCAGCCC | AGGAAGGTCAACCACTCACACA |
| β-actin | CGCCCCAGGCACCAGGGC | GGCTGGGGTGTTGAAGGT |

As a result, mutations were identified positively influencing the mRNA lifetime (deletion of C 1071, deletion of C 1079, deletion of T 1111, substitution of A for C 1144, deletion of A 1148, deletion of C 1155, deletion of A 1173, deletion of G 1183, deletion of C 1185, substitution of G for C 1536 and others). Said changes in various combinations of 4-10 mutations were brought together into synthetic constructs including a coding region of the VEGF gene and 3'UTR, and optimized in relation to the VEGF gene. The main of the obtained variants are presented by Seq#2-11. In order to select the most optimal variant from the developed gene constructs, a comparative study was carried out during which the accumulation dynamics of mRNA by transfected cells was determined by the real-time PCR as discussed above. Fig. 1 shows the main ten gene constructs as a multiple magnification of the mRNA concentration at 24 and 48 hours after transfection.

As shown in the graph, in some cases, the combinations of several mutations each separately having a positive influence on the mRNA lifetime produced no significant increase in the accumulation of mRNA as compared to the original gene construct (Seq#1). For the gene constructs characterized by an increased mRNA lifetime, the lack of drop in concentration was observed only for Seq#11 while in other cases, the mRNA level dropped 48 hours after transfection as compared to the level at 24 hours. In this connection, precisely this gene construct was selected as the most optimal and was subjected to further comparative studies.

Fig. 2 shows the result of the real-time PCR for Seq#11 in comparison with non-optimized sequence Seq#1. As can been seen in the graph, as early as after12 hours, the optimized construct had a level of mRNA in the VEGF gene exceeding the parameter value in the control group using the original plasmid DNA for transfection. Importantly, the dynamics of increase in the mRNA concentration in both groups coincided with a peak value at 24 hours after transfection. However, a gradual decrease in the mRNA level associated with a rapid biodegradation of molecules was observed only in case of the original gene construct whereas a "plateau" phase lasting at least 6 hours and followed by a smooth decrease in the mRNA concentration was observed in case of the optimized construct (Seq#11). At the same time, the difference between the groups in terms of this value was 190% at the latest time point.

The developed plasmid DNA carrying the VEGF gene was studied *in vitro* in order to quantify the production of the VEGF protein by HEK273 cells. The concentration of therapeutic protein in the cultural medium was determined using ELISA 6, 12, 24, 48, 72 and 96 hours after cell transfection. It appeared that the accumulated concentration of therapeutic protein in the cultural medium for cells transfected with the gene construct was far higher than the parameter value in the control groups including the groups with alternative gene constructs characterized by a non-optimized 3'UTR sequence of the VEGF gene and standard lifetime of the transgene mRNA (Fig. 3). Importantly, the maximum increase in the VEGF concentration in the cultural medium for both groups of transfected cells (2.3-2.4 times) was observed 36 hours after the experiment started, which agreed with the real-time PCR data showing a peak concentration of the mRNA in gene constructs at 24 hours. Later on, while the concentration of VEGF protein in the medium of cells transfected with the optimized gene construct continued to increase at a high rate, the increase significantly slowed down in the group with the original plasmid DNA and slightly exceeded the increase in the control group (non-transfected culture).

While the accumulated concentration of the VEGF protein measured in the cultural medium makes it possible to estimate its production by cells and to quantify its increase at each control time point, it does not allow the intracellular concentration of protein to be evaluated at each of the time points. At the same time, it is particularly important for the integrated assessment of the mRNA lifetime in gene constructs to determine the level of the VEGF protein in cells at the latest time points of observation. In this connection, the cells after 4 days of cultivation were subjected to the western blotting analysis according to a standard technique using anti-VEGF antibodies. The protein concentration was found to be the highest in the cell lyzate transfected with optimized gene construct Seq#11 (Fig. 4). At the same time, the protein level in case of cells transfected with the original construct was insignificantly higher than the endogenous concentration (control) at this stage of observation.

Moreover, the conditioned cell medium transfected with optimized gene constructs was characterized by a significantly higher angiogenic activity as determined by a routine test using HUVEC tube formation assay.

Comparing the findings of the conducted studies, one may conclude that mRNA in the optimized gene construct (Seq#11) was characterized by a longer lifetime leading to a more prolonged production of the VEGF therapeutic protein at high concentrations up to the latest points of observation. In contrast, the original gene construct was characterized by a lower mRNA stability, short and less marked biological effect.

Therefore, the main technical effect of the present invention as defined in the claims consists in increasing the total production of therapeutic protein due to an increased mRNA lifetime achieved by identifying the optimal 3'UTR sequences of the VEGF gene. It has been surprisingly found that substitution of just one nucleotide in the target region has such a marked effect on the mRNA lifetime and, accordingly, on the production of therapeutic protein. In all prior art researches in this field other changes were performed involving far more nucleotides. Considering that the functionality of each site in the mRNA's 3'UTR is insufficiently studied, precisely minimal, pointwise changes are the most favorable in terms of safety and leveling the influence on other aspects of the mRNA metabolism as long as such changes allow the necessary effect to be achieved. It is important to note that the length of the 3'UTR sequence in the VEGF gene is at least 250 nucleotides so that the total number of only single pointwise mutations in the form of substitution of one nucleotide with another is at least 750. Taking into account other possible variants of changes (deletions, duplications, insertions) and also allowing for involvement of more than one site, the number of gene construct variants differing in the 3'UTR of the VEGF gene amounts to hundreds of thousands. Therefore, identification of a number of variants of changes in the sequence resulting in an increased mRNA lifetime without negatively influencing other aspects of its functioning is equal to winning a jack-pot.

Relying on its experience in the development of preparations for gene therapy, the Applicant produced pharmaceutical compositions based on the developed optimized gene constructs and pharmaceutically acceptable adjuvants presented by at least one cryoprotectant with the filler properties, and a pH stabilizer, which in effective amounts enable preparation of an isotonic solution of plasmid DNA for injections.

The pharmaceutical composition has the following formulation enabling to preserve the properties of the optimized plasmid DNA:
- plasmid DNA: from 0.1 to 10 mg/ml, preferably from 0.5 to 4 mg/ml, most preferably from 0.8 to 1.2 mg/ml;
- glucose (dextrose): from 200 to 400 mM, preferably from 250 to 350 mM, most preferably from 280 mM to 320 mM;
- sodium phosphate (mixture of trisodium, disodium and monosodium phosphates) at a concentration of 3 to 30 mM, preferably from 5 to 20 mM, most preferably from 8 to 12 mM;
- solution pH: from 7.0 to 9.0, preferably from 7.2 to 8.5, most preferably from 7.4 to 8.2.

The pharmaceutical composition may have the following formulation:
- plasmid DNA according Seq#ll: 1.2 mg;
- dextrose monohydrate: 60 mg;
- sodium hydrogen phosphate dodecahydrate: 3.94 mg;
- sodium dihydrogen phosphate dehydrate: 0.16 mg.

Said composition was used in the studies described below in the examples.

The developed gene constructs and the pharmaceutical compositions based thereon were studied *in vivo* on experimental models reproducing the main pathological and pathomorphological symptoms indicative of the diseases known to be suitable for the application of the plasmid DNA with the VEGF gene.

### Example 1. Model of chronic ischemia of lower limbs

The study was performed using immunodeficient mice (n=90) with the right femoral artery having been cut across in the inguinal region for modeling chronic ischemia of lower limbs (CILL). Two modes were used to administer the pharmaceutical composition based on the plasmid DNA according to Seq#ll: 100 µg once on day 14 and 200 µg twice on days 1 and 14 to the proximal and distal portions of the post-surgical wound. As a control, three groups of animals were used: healthy animals; animals after surgery, receiving no substances; animals after surgery, receiving water for injection. The animals were withdrawn from the experiment on 7, 21, 35 days after surgery, the limbs were subjected to histological study to determine the number of vessels and the ratio of endotheliocytes to muscle fibers. On days 1, 7, 14, 21, 28 and 35, scanning laser dopplerometry was performed to determine the blood flow perfusion rate in the ischemic and healthy limbs.

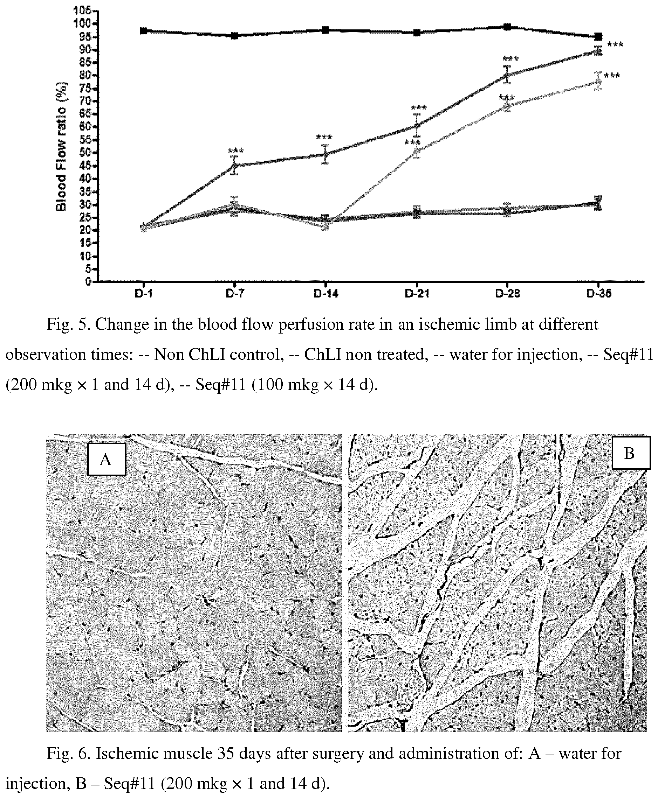

It appeared that regardless of the dose and mode of administration, the blood flow perfusion rate in the ischemic limb increased only in the experimental groups 7 days after the administration of the developed preparation for gene therapy. The blood flow perfusion rate was growing and by the end of observations it reached the values close to the parameter of healthy animals. In the control groups of animals after surgery, the blood flow perfusion rate remained at a low level with no positive dynamics (Fig. 5).

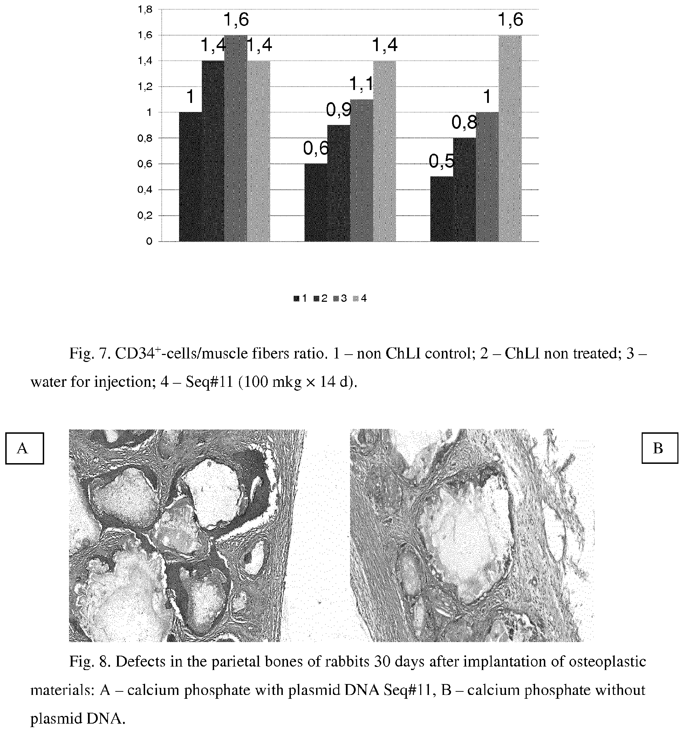

The functional results were confirmed by the histological study findings. More vessels have been identified in the experimental groups (Fig. 6) and also a higher ratio of CD34⁺-cells (endotheliocytes) to the muscle fiber (Fig. 7).

### Example 2. Model of a critical-sized bone defect

The developed gene construct according to Seq#11 was combined with a calcium phosphate matrix according to the predetermined protocol.
1. Preparation of carrier:
   a) ablution (incubation in 0.5 M phosphate buffer in 1 ml at 37°C with continuous shaking for 12 hours);
   b) equilibration (treatment with 10 mM phosphate buffer in 1 ml at 37°C with continuous shaking, 3 times for 10 min);
   c) drying (incubation at 37°C until complete drying for 3 hours).
2. Application of a nucleic acid (incubation with a solution of plasmid DNA according to Seq#11 in 10 mM phosphate buffer at a concentration of 1 µg/µl at 37°C with continuous shaking for 12 hours).
3. Treatment of the resulting "carrier-gene construct" complex:
   a) ablution (treatment with 5 mM phosphate solution in 1 ml 3 times);
   b) drying (incubation at 37°C until complete drying for 3 hours).

The resulting gene-activated bone graft was studied in orthotopic conditions. The study was performed on Chinchilla rabbits (n=15). In each animal, two identical symmetrical full-thickness defects of both parietal bones with a diameter of 10 mm each were performed, which are "critical" to the rabbits because the natural recovery process never ends in a complete consolidation without optimizing influences. A gene-activated osteoplastic material (experimental group) consisting of calcium phosphate and the plasmid DNA according to Seq#11 was implanted in the defects of right parietal bones, and a carrier without the plasmid DNA (control group) was implanted in the defects of left parietal bones. The animals were withdrawn from the experiment on days 30, 60 and 90, and the results were assessed using computer tomography and histological methods.

Due to an originally high density of the selected carrier (about 1800 HU) and the period of its bioresorption (more than 6 months), no objective assessment of the evidence of reparative osteogenesis was possible in comparative terms. However, according to the histological study, the evidence of osteogenesis was observed in the central part of the defect already 30 days after surgery only where the gene-activated osteoplastic material was used (Fig. 8).

As seen in Fig. 8, the material granules in the central part of the defect in the form of a loose fibrous connective tissue were the source of reparative osteogenesis whereas in the control defect, calcium phosphate without the plasmid DNA was surrounded only by a loose fibrous connective tissue without any evidence of the osseous tissue formation. The obtained data is indicative of that the plasmid DNA according to Seq#11 administered to the area of the critical-sized bone defect resulted in a marked induction of reparative osteogenesis, which may be associated both with the angiogenesis-mediated effect and the direct influence of the VEGF on the mesenchymal cell line.
Therefore, the above examples illustrate the fact that the developed optimized gene construct and the pharmaceutical composition based thereon have a marked angiogenic activity so that it may be hopefully used for the treatment of ischemic diseases of the cardiovascular system. In addition, the optimized gene construct with the VEGF gene may be efficient for the treatment of other pathological conditions requiring activation of a reparative process, such as injuries of skin and locomotor apparatus, injuries of peripheral nerves, diabetic foot syndrome, and amyotrophic lateral sclerosis.

### SEQUENCE LISTING

<110> "NextGen" Company Limited
<120> NUCLEOTIDE SEQUENCE AND PHARMACEUTICAL COMPOSITION BASED THEREON WITH PROLONGED VEGF TRANSGENE EXPRESSION
<130> NCOL004PEP
<140> 15893474.5
<141> 2015-12-07
<150> RU20150119768
<151> 2015-05-26
<150> PCT/RU2015/000852
<151> 2015-12-07
<160> 11
<170> PatentIn version 3.5
   Seq#1. The sequence of the modified base genetic structure. Point of reference \226 the first nucleotide. 
1 tatgccaagt acgcccccta ttgacgtcaa tgacggtaaa tggcccgcct ggcattatgc
61 ccagtacatg accttatggg actttcctac ttggcagtac atctacgtat tagtcatcgc
121 tattaccatg gtgatgcggt tttggcagta catcaatggg cgtggatagc ggtttgactc
181 acggggattt ccaagtctcc accccattga cgtcaatggg agtttgtttt ggcaccaaaa
241 tcaacgggac tttccaaaat gtcgtaacaa ctccgcccca ttgacgcaaa tgggcggtag
301 gcgtgtacgg tgggaggtct atataagcag agctggttta gtgaaccgtc agatccgcta
361 gtggatccaa agaattcggg cctccgaaac catgaacttt ctgctgtctt gggtgcattg
421 gagccttgcc ttgctgctct acctccacca tgccaagtgg tcccaggctg cacccatggc
481 agaaggagga gggcagaatc atcacgaagt ggtgaagttc atggatgtct atcagcgcag
541 ctactgccat ccaatcgaga ccctggtgga catcttccag gagtaccctg atgagatcga
601 gtacatcttc aagccatcct gtgtgcccct gatgcgatgc gggggctgct gcaatgacga
661 gggcctggag tgtgtgccca ctgaggagtc caacatcacc atgcagatta tgcggatcaa
721 acctcaccaa ggccagcaca taggagagat gagcttccta cagcacaaca aatgtgaatg
781 cagaccaaag aaagatagag caagacaaga aaatccctgt gggccttgct cagagcggag
841 aaagcatttg tttgtacaag atccgcagac gtgtaaatgt tcctgcaaaa acacagactc
901 gcgttgcaag gcgaggcagc ttgagttaaa cgaacgtact tgcagatgtg acaagccgag
961 gcggtgaccc gggtggggta ccaggtaagt gtacccaatt cgccctatag tgagtcgtat
1021 tacaattcac tggccgtcgt tttacaacgt cgtgactggg aaaaccctgg cgttacccca
1081 acttaatcgc cttgcagcac atcccccttt tcgccagctg gcgtaatagc gaagaggccc
1141 gcaacgaatc gccccttccc aacagttgcg caagcctgaa tggccgaatg gagatccaat
1201 ttttaagtgt ntaatgtgtt aaactactga ttctaattgt ttgtgtatnt tagattcaca
1261 gtcccaaggc tcatttcagg cccctcagtc ctcacagtct gttcatgatc ataatcagcc
1321 ataccacatt tgtagaggtt ttacttgctt taaaaaacct cccacacctc cccctgaacc
1381 tgaaacataa aatgaatgca attgttgttg ttaacttgtt tattgcagct tataatggtt
1441 acaaataaag caatagcatc acaaatttca caaataaagc atttttttca ctgcattcta
1501 gttgtggttt gtccaaactc atcaatgtat cttaaggcgt aaattgtaag cgttaatatt
1561 ttgttaaaat tcgcgttaaa tttttgttaa atcagctcat tttttaacca ataggccgaa
1621 atcggcaaaa tcccttataa atcaaaagaa tagaccgaga tagggttgag tgttgttcca
1681 gtttggaaca agagtccact attaaagaac gtggactcca acgtcaaagg gcgaaaaacc
1741 gtctatcagg gcgatggccc actacgtgaa ccatcaccct aatcaagttt tttggggtcg
1801 aggtgccgta aagcactaaa tcggaaccct aaagggagcc cccgatttag agcttgacgg
1861 ggaaagccgg cgaacgtggc gagaaaggaa gggaagaaag cgaaaggagc gggcgctagg
1921 gcgctggcaa gtgtagcggt cacgctgcgc gtaaccacca cacccgccgc gcttaatgcg
1981 ccgctacagg gcgcgtcagg tggcactttt cggggaaatg tgcgcggaac ccctatttgt
2041 ttatttttct aaatacattc aaatatgtat ccgctcatga gacaataacc ctgataaatg
2101 cttcaataat attgaaaaag gaagagtcct gaggcggaaa gaaccagctg tggaatgtgt
 
2161 gtcagttagg gtgtggaaag tccccaggct ccccagcagg cagaagtatg caaagcatgc
2221 atctcaatta gtcagcaacc aggtgtggaa agtccccagg ctccccagca ggcagaagta
2281 tgcaaagcat gcatctcaat tagtcagcaa ccatagtccc gcccctaact ccgcccatcc
2341 cgcccctaac tccgcccagt tccgcccatt ctccgcccca tggctgacta atttttttta
2401 tttatgcaga ggccgaggcc gcctcggcct ctgagctatt ccagaagtag tgaggaggct
2461 tttttggagg cctaggcttt tgcaaagatc gatcaagaga caggatgagg atcgtttcgc
2521 atgattgaac aagatggatt gcacgcaggt tctccggccg cttgggtgga gaggctattc
2581 ggctatgact gggcacaaca gacaatcggc tgctctgatg ccgccgtgtt ccggctgtca
2641 gcgcaggggc gcccggttct ttttgtcaag accgacctgt ccggtgccct gaatgaactg
2701 caagacgagg cagcgcggct atcgtggctg gccacgacgg gcgttccttg cgcagctgtg
2761 ctcgacgttg tcactgaagc gggaagggac tggctgctat tgggcgaagt gccggggcag
2821 gatctcctgt catctcacct tgctcctgcc gagaaagtat ccatcatggc tgatgcaatg
2881 cggcggctgc atacgcttga tccggctacc tgcccattcg accaccaagc gaaacatcgc
2941 atcgagcgag cacgtactcg gatggaagcc ggtcttgtcg atcaggatga tctggacgaa
3001 gagcatcagg ggctcgcgcc agccgaactg ttcgccaggc tcaaggcgag catgcccgac
3061 ggcgaggatc tcgtcgtgac ccatggcgat gcctgcttgc cgaatatcat ggtggaaaat
3121 ggccgctttt ctggattcat cgactgtggc cggctgggtg tggcggaccg ctatcaggac
3181 atagcgttgg ctacccgtga tattgctgaa gagcttggcg gcgaatgggc tgaccgcttc
3241 ctcgtgcttt acggtatcgc cgctcccgat tcgcagcgca tcgccttcta tcgccttctt
3301 gacgagttct tctgagcggg actctggggt tcgaaatgac cgaccaagcg acgcccaacc
3361 tgccatcacg agatttcgat tccaccgccg ccttctatga aaggttgggc ttcggaatcg
3421 ttttccggga cgccggctgg atgatcctcc agcgcgggga tctcatgctg gagttcttcg
3481 cccaccctag ggggaggcta actgaaacac ggaaggagac aataccggaa ggaacccgcg
3541 ctatgacggc aataaaaaga cagaataaaa cgcacggtgt tgggtcgttt gttcataaac
3601 gcggggttcg gtcccagggc tggcactctg tcgatacccc accgagaccc cattggggcc
3661 aatacgcccg cgtttcttcc ttttccccac cccacccccc aagttcgggt gaaggcccag
3721 ggctcgcagc caacgtcggg gcggcaggcc ctgccatagc ctcaggttac tcatatatac
3781 tttagattga tttaaaactt catttttaat ttaaaaggat ctaggtgaag atcctttttg
3841 ataatctcat gaccaaaatc ccttaacgtg agttttcgtt ccactgagcg tcagaccccg
3901 tagaaaagat caaaggatct tcttgagatc ctttttttct gcgcgtaatc tgctgcttgc
3961 aaacaaaaaa accaccgcta ccagcggtgg tttgtttgcc ggatcaagag ctaccaactc
4021 tttttccgaa ggtaactggc ttcagcagag cgcagatacc aaatactgtt cttctagtgt
4081 agccgtagtt aggccaccac ttcaagaact ctgtagcacc gcctacatac ctcgctctgc
4141 taatcctgtt accagtggct gctgccagtg gcgataagtc gtgtcttacc gggttggact
4201 caagacgata gttaccggat aaggcgcagc ggtcgggctg aacggggggt tcgtgcacac
4261 agcccagctt ggagcgaacg acctacaccg aactgagata cctacagcgt gagctatgag
4321 aaagcgccac gcttcccgaa gggagaaagg cggacaggta tccggtaagc ggcagggtcg
4381 gaacaggaga gcgcacgagg gagcttccag ggggaaacgc ctggtatctt tatagtcctg
4441 tcgggtttcg ccacctctga cttgagcgtc gatttttgtg atgctcgtca ggggggcgga
4501 gcctatggaa aaacgccagc aacgcggcct ttttacggtt cctggccttt tgctggcctt
4561 ttgctcacat gttctttcct gcgttatccc ctgattctgt ggataaccgt attaccgcca
4621 tgcattagtt attaatagta atcaattacg gggtcattag ttcatagccc atatatggag
4681 ttccgcgtta cataacttac ggtaaatggc ccgcctggct gaccgcccaa cgacccccgc
4741 ccattgacgt caataatgac gtatgttccc atagtaacgc caatagggac tttccattga
4801 cgtcaatggg tggagtattt acggtaaact gcccacttgg cagtacatca agtgtatca
 Seq2. Optimized sequence in 1070 \226 1600 range with following modifications: 1071 ñ deletion, 1079 ñ deletion, 1144 a to c substitution, 1148 a deletion. 
1070 ggttacccaa cttaatcgcc ttgcagcaca tccccctttt cgccagctgg cgtaatagcg
1131 aagaggcccg cañcgatcgc cccttcccaa cagttgcgca agcctgaatg gccgaatgga
1191 gatccaattt ttaagtgtnt aatgtgttaa actactgatt ctaattgttt gtgtatntta
1251 gattcacagt cccaaggctc atttcaggcc cctcagtcct cacagtctgt tcatgatcat
1311 aatcagccat accacatttg tagaggtttt acttgcttta aaaaacctcc cacacctccc
1371 cctgaacctg aaacataaaa tgaatgcaat tgttgttgtt aacttgttta ttgcagctta
1431 taatggttac aaataaagca atagcatcac aaatttcaca aataaagcat ttttttcact
1491 gcattctagt tgtggtttgt ccaaactcat caatgtatct taaggcgtaa attgtaagcg
1551 ttaatatttt gttaaaattc gcgttaaatt tttgttaaat cagctcat
 Seq#3. Optimized sequence in 1070 \226 1600 range with following modifications: 1071 c deletion, 1079 c deletion, 1111 t deletion, 1144 a to c substitution, 1148 a deletion. 
1070 ggttacccaa cttaatcgcc ttgcagcaca tccccctttc gccagctggc
gtaatagcga
 
1131 agaggcccgc accgatcgcc ccttcccaac agttgcgcaa gcctgaatgg ccgaatggag
1191 atccaatttt taagtgtnta atgtgttaaa ctactgattc taattgtttg tgtatnttag
1251 attcacagtc ccaaggctca tttcaggccc ctcagtcctc acagtctgtt catgatcata
1311 atcagccata ccacatttgt agaggtttta cttgctttaa aaaacctccc acacctcccc
1371 ctgaacctga aacataaaat gaatgcaatt gttgttgtta acttgtttat tgcagcttat
1431 aatggttaca aataaagcaa tagcatcaca aatttcacaa ataaagcatt tttttcactg
1491 cattctagtt gtggtttgtc caaactcatc aatgtatctt aaggcgtaaa ttgtaagcgt
1551 taatattttg ttaaaattcg cgttaaattt ttgttaaatc agctcat
 Seq#4. Optimized sequence in 1070 \226 1600 range with following modifications: 1071 c deletion, 1111 t deletion, 1148 a deletion, 1155 c deletion. 
\205\205.1070 ggttacccca acttaatcgc cttgcagcac atcccccttt cgccagctgg
cgtaatagcg
 1131 aagaggcccg caacgatcgc ccttcccaac agttgcgcaa gcctgaatgg ccgaatggag
 1191 atccaatttt taagtgtnta atgtgttaaa ctactgattc taattgtttg tgtatnttag
 1251 attcacagtc ccaaggctca tttcaggccc ctcagtcctc acagtctgtt catgatcata
 1311 atcagccata ccacatttgt agaggtttta cttgctttaa aaaacctccc acacctcccc
 1371 ctgaacctga aacataaaat gaatgcaatt gttgttgtta acttgtttat tgcagcttat
 1431 aatggttaca aataaagcaa tagcatcaca aatttcacaa ataaagcatt tttttcactg
 1491 cattctagtt gtggtttgtc caaactcatc aatgtatctt aaggcgtaaa ttgtaagcgt
 1551 taatattttg ttaaaattcg cgttaaattt ttgttaaatc agctcat
 Seq#5. Optimized sequence in 1070 \226 1600 range with following modifications: 1071 c deletion, 1079 c deletion, 1148 a deletion, 1155 c deletion, 1173 a deletion. 
1070 ggttacccaa cttaatcgcc ttgcagcaca tccccctttt cgccagctgg
cgtaatagcg
1131 aagaggcccg caacgatcgc ccttcccaac agttgcgcag cctgaatggc cgaatggaga
1191 tccaattttt aagtgtntaa tgtgttaaac tactgattct aattgtttgt gtatnttaga
1251 ttcacagtcc caaggctcat ttcaggcccc tcagtcctca cagtctgttc atgatcataa
1311 tcagccatac cacatttgta gaggttttac ttgctttaaa aaacctccca cacctccccc
1371 tgaacctgaa acataaaatg aatgcaattg ttgttgttaa cttgtttatt gcagcttata
1431 atggttacaa ataaagcaat agcatcacaa atttcacaaa taaagcattt ttttcactgc
1491 attctagttg tggtttgtcc aaactcatca atgtatctta aggcgtaaat tgtaagcgtt
1551 aatattttgt taaaattcgc gttaaatttt tgttaaatca gctcat
 Seq#6. Optimized sequence in 1070 \226 1600 range with following modifications: 1079 c deletion, 1144 a to c substitution, 1155 c deletion, 1173 a deletion, 1183 g deletion. 
1070 gcgttaccca acttaatcgc cttgcagcac atcccccttt tcgccagctg
gcgtaatagc
1131 gaagaggccc gcaccgatcg cccttcccaa cagttgcgca gcctgaatgg cgaatggag
1191 atccaatttt taagtgtnta atgtgttaaa ctactgattc taattgtttg tgtatnttag
1251 attcacagtc ccaaggctca tttcaggccc ctcagtcctc acagtctgtt catgatcata
1311 atcagccata ccacatttgt agaggtttta cttgctttaa aaaacctccc acacctcccc
1371 ctgaacctga aacataaaat gaatgcaatt gttgttgtta acttgtttat tgcagcttat
1431 aatggttaca aataaagcaa tagcatcaca aatttcacaa ataaagcatt tttttcactg
1491 cattctagtt gtggtttgtc caaactcatc aatgtatctt aaggcgtaaa ttgtaagcgt
1551 taatattttg ttaaaattcg cgttaaattt ttgttaaatc agctcat
 Seq#7. Optimized sequence in 1070 \226 1600 range with following modifications: 1111 t deletion, 1148 a deletion, 1183 g deletion, 1185 c deletion, 1536 g to c substituion. 
1070 gcgttacccc aacttaatcg ccttgcagca catccccctt tcgccagctg
gcgtaatagc
1131 gaagaggccc gcaacgatcg ccccttccca acagttgcgc aagcctgaat gcgaatggag
1191 atccaatttt taagtgtnta atgtgttaaa ctactgattc taattgtttg tgtatnttag
1251 attcacagtc ccaaggctca tttcaggccc ctcagtcctc acagtctgtt catgatcata
1311 atcagccata ccacatttgt agaggtttta cttgctttaa aaaacctccc acacctcccc
1371 ctgaacctga aacataaaat gaatgcaatt gttgttgtta acttgtttat tgcagcttat
1431 aatggttaca aataaagcaa tagcatcaca aatttcacaa ataaagcatt tttttcactg
1491 cattctagtt gtggtttgtc caaactcatc aatgtatctt aacgcgtaaa ttgtaagcgt
 1551 taatattttg ttaaaattcg cgttaaattt ttgttaaatc agctcat Seq#8. Optimized sequence in 1070 \226 1600 range with following modifications: 1111 t deletion, 1173 a deletion, 1183 g deletion, 1185 c deletion, 1536 g to c substitution. 
1070  gcgttacccc aacttaatcg ccttgcagca catccccctt tcgccagctg
gcgtaatagc
1131 gaagaggccc gcaacgaatc gccccttccc aacagttgcg cagcctgaat gcgaatggag
1191 atccaatttt taagtgtnta atgtgttaaa ctactgattc taattgtttg tgtatnttag
1251 attcacagtc ccaaggctca tttcaggccc ctcagtcctc acagtctgtt catgatcata
1311 atcagccata ccacatttgt agaggtttta cttgctttaa aaaacctccc acacctcccc
1371 ctgaacctga aacataaaat gaatgcaatt gttgttgtta acttgtttat tgcagcttat
1431 aatggttaca aataaagcaa tagcatcaca aatttcacaa ataaagcatt tttttcactg
1491 cattctagtt gtggtttgtc caaactcatc aatgtatctt aacgcgtaaa ttgtaagcgt
1551 taatattttg ttaaaattcg cgttaaattt ttgttaaatc agctcat
 Seq#9. Optimized sequence in 1070 \226 1600 range with following modifications: 1079 c deletion, 1111 t deletion, 1155 c deletion, 1183 g deletion, 1185 c deletion, 1536 g to c substitution. 
1070 gcgttaccca acttaatcgc cttgcagcac atcccccttt cgccagctgg
cgtaatagcg
1131 aagaggcccg caacgaatcg cccttcccaa cagttgcgca agcctgaatg cgaatggaga
1191 tccaattttt aagtgtntaa tgtgttaaac tactgattct aattgtttgt gtatnttaga
1251 ttcacagtcc caaggctcat ttcaggcccc tcagtcctca cagtctgttc atgatcataa
1311 tcagccatac cacatttgta gaggttttac ttgctttaaa aaacctccca cacctccccc
1371 tgaacctgaa acataaaatg aatgcaattg ttgttgttaa cttgtttatt gcagcttata
1431 atggttacaa ataaagcaat agcatcacaa atttcacaaa taaagcattt ttttcactgc
1491 attctagttg tggtttgtcc aaactcatca atgtatctta acgcgtaaat tgtaagcgtt
1551 aatattttgt taaaattcgc gttaaatttt tgttaaatca gctcat
 Seq#10. Optimized sequence in 1070 \226 1600 range with following modifications: 1071 c deletion, 1079 c deletion, 1111 t deletion, 1144 a to c substitution, 1148 a deletion, 1155 c deletion, 1173 a deletion, 1183 g deletion, 1185 c deletion, 1536 g to c substitution. 
1070 ggttacccaa cttaatcgcc ttgcagcaca tccccctttc gccagctggc
gtaatagcga
1131 agaggcccgc accgatcgcc cttcccaaca gttgcgcagc ctgaatgcga atggagatcc
1191 aatttttaag tgtntaatgt gttaaactac tgattctaat tgtttgtgta tnttagattc
1251 acagtcccaa ggctcatttc aggcccctca gtcctcacag tctgttcatg atcataatca
1311 gccataccac atttgtagag gttttacttg ctttaaaaaa cctcccacac ctccccctga
1371 acctgaaaca taaaatgaat gcaattgttg ttgttaactt gtttattgca gcttataatg
1431 gttacaaata aagcaatagc atcacaaatt tcacaaataa agcatttttt tcactgcatt
1491 ctagttgtgg tttgtccaaa ctcatcaatg tatcttaacg cgtaaattgt aagcgttaat
1551 attttgttaa aattcgcgtt aaatttttgt taaatcagct cat
 Seq#11. Optimized sequence with following modifications: 1079 c deletion, 1111 t deletion, 1144 a to c substitution, 1148 a deletion, 1155 c deletion, 1173 a deletion, 1185 c deletion, 1536 g to c substitution.

1 tatgccaagt acgcccccta ttgacgtcaa tgacggtaaa tggcccgcct ggcattatgc
61 ccagtacatg accttatggg actttcctac ttggcagtac atctacgtat tagtcatcgc
121 tattaccatg gtgatgcggt tttggcagta catcaatggg cgtggatagc ggtttgactc
181 acggggattt ccaagtctcc accccattga cgtcaatggg agtttgtttt ggcaccaaaa
241 tcaacgggac tttccaaaat gtcgtaacaa ctccgcccca ttgacgcaaa tgggcggtag
301 gcgtgtacgg tgggaggtct atataagcag agctggttta gtgaaccgtc agatccgcta
361 gtggatccaa agaattcggg cctccgaaac catgaacttt ctgctgtctt gggtgcattg
421 gagccttgcc ttgctgctct acctccacca tgccaagtgg tcccaggctg cacccatggc
481 agaaggagga gggcagaatc atcacgaagt ggtgaagttc atggatgtct atcagcgcag
541 ctactgccat ccaatcgaga ccctggtgga catcttccag gagtaccctg atgagatcga
601 gtacatcttc aagccatcct gtgtgcccct gatgcgatgc gggggctgct gcaatgacga
661 gggcctggag tgtgtgccca ctgaggagtc caacatcacc atgcagatta tgcggatcaa
721 acctcaccaa ggccagcaca taggagagat gagcttccta cagcacaaca aatgtgaatg
781 cagaccaaag aaagatagag caagacaaga aaatccctgt gggccttgct cagagcggag
841 aaagcatttg tttgtacaag atccgcagac gtgtaaatgt tcctgcaaaa acacagactc

901 gcgttgcaag gcgaggcagc ttgagttaaa cgaacgtact tgcagatgtg acaagccgag
961 gcggtgaccc gggtggggta ccaggtaagt gtacccaatt cgccctatag tgagtcgtat
1021 tacaattcac tggccgtcgt tttacaacgt cgtgactggg aaaaccctgg cgttacccaa
1081 cttaatcgcc ttgcagcaca tccccctttc gccagctggc gtaatagcga agaggcccgc
1141 accgatcgcc cttcccaaca gttgcgcagc ctgaatggcg aatggagatc caatttttaa
1201 gtgtataatg tgttaaacta ctgattctaa ttgtttgtgt attttagatt cacagtccca
1261 aggctcattt caggcccctc agtcctcaca gtctgttcat gatcataatc agccatacca
1321 catttgtaga ggttttactt gctttaaaaa acctcccaca cctccccctg aacctgaaac
1381 ataaaatgaa tgcaattgtt gttgttaact tgtttattgc agcttataat ggttacaaat
1441 aaagcaatag catcacaaat ttcacaaata aagcattttt ttcactgcat tctagttgtg
1501 gtttgtccaa actcatcaat gtatcttaac gcgtaaattg taagcgttaa tattttgtta
1561 aaattcgcgt taaatttttg ttaaatcagc tcatttttta accaataggc cgaaatcggc
1621 aaaatccctt ataaatcaaa agaatagacc gagatagggt tgagtgttgt tccagtttgg
1681 aacaagagtc cactattaaa gaacgtggac tccaacgtca aagggcgaaa aaccgtctat
1741 cagggcgatg gcccactacg tgaaccatca ccctaatcaa gttttttggg gtcgaggtgc
1801 cgtaaagcac taaatcggaa ccctaaaggg agcccccgat ttagagcttg acggggaaag
1861 ccggcgaacg tggcgagaaa ggaagggaag aaagcgaaag gagcgggcgc tagggcgctg
1921 gcaagtgtag cggtcacgct gcgcgtaacc accacacccg ccgcgcttaa tgcgccgcta
1981 cagggcgcgt caggtggcac ttttcgggga aatgtgcgcg gaacccctat ttgtttattt
2041 ttctaaatac attcaaatat gtatccgctc atgagacaat aaccctgata aatgcttcaa
2101 taatattgaa aaaggaagag tcctgaggcg gaaagaacca gctgtggaat gtgtgtcagt
2161 tagggtgtgg aaagtcccca ggctccccag caggcagaag tatgcaaagc atgcatctca
2221 attagtcagc aaccaggtgt ggaaagtccc caggctcccc agcaggcaga agtatgcaaa
2281 gcatgcatct caattagtca gcaaccatag tcccgcccct aactccgccc atcccgcccc
2341 taactccgcc cagttccgcc cattctccgc cccatggctg actaattttt tttatttatg
2401 cagaggccga ggccgcctcg gcctctgagc tattccagaa gtagtgagga ggcttttttg
2461 gaggcctagg cttttgcaaa gatcgatcaa gagacaggat gaggatcgtt tcgcatgatt
2521 gaacaagatg gattgcacgc aggttctccg gccgcttggg tggagaggct attcggctat
2581 gactgggcac aacagacaat cggctgctct gatgccgccg tgttccggct gtcagcgcag
2641 gggcgcccgg ttctttttgt caagaccgac ctgtccggtg ccctgaatga actgcaagac
2701 gaggcagcgc ggctatcgtg gctggccacg acgggcgttc cttgcgcagc tgtgctcgac
2761 gttgtcactg aagcgggaag ggactggctg ctattgggcg aagtgccggg gcaggatctc
2821 ctgtcatctc accttgctcc tgccgagaaa gtatccatca tggctgatgc aatgcggcgg
2881 ctgcatacgc ttgatccggc tacctgccca ttcgaccacc aagcgaaaca tcgcatcgag
2941 cgagcacgta ctcggatgga agccggtctt gtcgatcagg atgatctgga cgaagagcat
3001 caggggctcg cgccagccga actgttcgcc aggctcaagg cgagcatgcc cgacggcgag
3061 gatctcgtcg tgacccatgg cgatgcctgc ttgccgaata tcatggtgga aaatggccgc
3121 ttttctggat tcatcgactg tggccggctg ggtgtggcgg accgctatca ggacatagcg
3181 ttggctaccc gtgatattgc tgaagagctt ggcggcgaat gggctgaccg cttcctcgtg
3241 ctttacggta tcgccgctcc cgattcgcag cgcatcgcct tctatcgcct tcttgacgag
3301 ttcttctgag cgggactctg gggttcgaaa tgaccgacca agcgacgccc aacctgccat
3361 cacgagattt cgattccacc gccgccttct atgaaaggtt gggcttcgga atcgttttcc
3421 gggacgccgg ctggatgatc ctccagcgcg gggatctcat gctggagttc ttcgcccacc
3481 ctagggggag gctaactgaa acacggaagg agacaatacc ggaaggaacc cgcgctatga
3541 cggcaataaa aagacagaat aaaacgcacg gtgttgggtc gtttgttcat aaacgcgggg
3601 ttcggtccca gggctggcac tctgtcgata ccccaccgag accccattgg ggccaatacg
3661 cccgcgtttc ttccttttcc ccaccccacc ccccaagttc gggtgaaggc ccagggctcg
3721 cagccaacgt cggggcggca ggccctgcca tagcctcagg ttactcatat atactttaga
3781 ttgatttaaa acttcatttt taatttaaaa ggatctaggt gaagatcctt tttgataatc
3841 tcatgaccaa aatcccttaa cgtgagtttt cgttccactg agcgtcagac cccgtagaaa
3901 agatcaaagg atcttcttga gatccttttt ttctgcgcgt aatctgctgc ttgcaaacaa
3961 aaaaaccacc gctaccagcg gtggtttgtt tgccggatca agagctacca actctttttc
4021 cgaaggtaac tggcttcagc agagcgcaga taccaaatac tgttcttcta gtgtagccgt
4081 agttaggcca ccacttcaag aactctgtag caccgcctac atacctcgct ctgctaatcc
4141 tgttaccagt ggctgctgcc agtggcgata agtcgtgtct taccgggttg gactcaagac
4201 gatagttacc ggataaggcg cagcggtcgg gctgaacggg gggttcgtgc acacagccca
4261 gcttggagcg aacgacctac accgaactga gatacctaca gcgtgagcta tgagaaagcg
4321 ccacgcttcc cgaagggaga aaggcggaca ggtatccggt aagcggcagg gtcggaacag
4381 gagagcgcac gagggagctt ccagggggaa acgcctggta tctttatagt cctgtcgggt
4441 ttcgccacct ctgacttgag cgtcgatttt tgtgatgctc gtcagggggg cggagcctat
4501 ggaaaaacgc cagcaacgcg gcctttttac ggttcctggc cttttgctgg ccttttgctc
4561 acatgttctt tcctgcgtta tcccctgatt ctgtggataa ccgtattacc gccatgcatt
4621 agttattaat agtaatcaat tacggggtca ttagttcata gcccatatat ggagttccgc

4681 gttacataac ttacggtaaa tggcccgcct ggctgaccgc ccaacgaccc ccgcccattg
4741 acgtcaataa tgacgtatgt tcccatagta acgccaatag ggactttcca ttgacgtcaa
4801 tgggtggagt atttacggta aactgcccac ttggcagtac atcaagtgta tca

## Claims

1. A screening method for VEGF mRNAs with extended lifetime, said method comprising the following steps:
i) performing pointwise deletions in the 3'-untranslated region (3'-UTR)of a VEGF gene, wherein the deleted nucleotide is either not substituted or substituted with cytosine at the substitution points of guanine and adenine, to produce a polynucleotide comprising said modified 3'-UTR sequence;
ii) transcribing said polynucleotide into a mRNA transcript;
iii) determining the mRNA lifetime of said mRNA transcript after each deletion;
iv) analyzing said mRNA lifetime information.

2. The method according to claim 1, wherein the mRNA's 3'-untranslated region is an untranslated region of the gene selected from VEGF-121, VEGF-145, VEGF-165, VEGF-185 and other possible isoforms of the vascular endothelial growth factor gene.

3. A plasmid DNA comprising SEQ ID NO: 11.

4. mRNA encoded by the plasmid DNA according to claim 3.

5. A pharmaceutical composition for the regeneration of connective, muscular, neural tissues, comprising the plasmid DNA according to claim 3 and at least one cryoprotectant and pH stabilizer.

6. Use of the plasmid DNA according to claim 3 for the *in-vitro* or *ex-vivo* regeneration of connective, neural, muscular, osseous tissues and cardiovascular system tissues.

## Patentansprüche

1. Screening-Verfahren für VEGF-mRNAs mit verlängerter Lebensdauer, wobei das Verfahren die folgenden Schritte umfasst:
i) Durchführen punktueller Deletionen in der nicht translatierten 3'-Region (3'-UTR) eines VEGF-Gens, wobei das deletierte Nukleotid an den Substitutionsstellen von Guanin und Adenin entweder nicht substituiert oder mit Cytosin substituiert ist, um ein Polynukleotid zu erzeugen, das die modifizierte 3'-UTR-Sequenz umfasst;
ii) Transkribieren des Polynukleotids in ein mRNA-Transkript;
iii) Bestimmen der mRNA-Lebensdauer des mRNA-Transkripts nach jeder Deletion;
iv) Analysieren der mRNA-Lebensdauerinformation.

2. Verfahren nach Anspruch 1, wobei die nicht translatierte 3'-Region der mRNA eine nicht translatierte Region eines Gens ist, ausgewählt aus VEGF-121, VEGF-145, VEGF-165, VEGF-185 und anderen möglichen Isoformen des Gens des vaskulären endothelialen Wachstumsfaktors.

3. Plasmid-DNA, umfassend SEQ ID Nr.: 11.

4. mRNA, die von der Plasmid-DNA gemäß Anspruch 3 kodiert wird.

5. Pharmazeutische Zusammensetzung für die Regeneration von Binde-, Muskel- und Nervengeweben, umfassend die Plasmid-DNA gemäß Anspruch 3 und mindestens ein Kryoschutzmittel und einen pH-Stabilisator.

6. Verwendung der Plasmid-DNA gemäß Anspruch 3 für die *invitro-* oder *ex-vivo*-Regeneration von Bindegewebe, Nervengewebe, Muskelgewebe, Knochengewebe und Geweben des kardiovaskulären Systems.

## Revendications

1. Procédé de criblage d'ARNm de VEGF avec durée de vie prolongée, ledit procédé comprenant les étapes suivantes :
i) conduite de délétions ponctuelles dans la région non traduite en 3' (3'-UTR)d'un gène VEGF, dans lequel le nucléotide délété est soit non substitué ou substitué par cytosine aux points de substitution de guanine et d'adénine, pour produire un polynucléotide comprenant ladite séquence 3'-UTR modifiée ;
ii) transcription dudit polynucléotide en produit de transcription ARNm ;
iii) détermination de la durée de vie d'ARNm dudit produit de transcription ARNm après chaque délétion ;
iv) analyse desdites informations de durée de vie d'ARNm.

2. Procédé selon la revendication 1, dans lequel la région non traduite en 3' de l'ARNm est une région non traduite du gène choisi parmi VEGF-121, VEGF-145, VEGF-165, VEGF-185 et d'autres isoformes possibles du gène de facteur de croissance endothélial vasculaire.

3. ADN plasmidique comprenant SEQ ID NO : 11.

4. ARNm codé par l'ADN plasmidique selon la revendication 3.

5. Composition pharmaceutique pour la régénération de tissus conjonctif, musculaire, neuronal, comprenant l'ADN plasmidique selon la revendication 3 et au moins un cryoprotecteur et un stabilisant de pH.

6. Utilisation de l'ADN plasmidique selon la revendication 3 pour la régénération in *vitro* ou ex *vivo* de tissus conjonctif, neuronal, musculaire, osseux et du système cardiovasculaire.
